(19) Europäisches Patentamt
European Patent Office
Office européen des brevets

(11) Publication number: **0 102 688**
**B1**

(12) # EUROPEAN PATENT SPECIFICATION

(45) Date of publication of patent specification: **06.08.86**

(51) Int. Cl.[4]: **C 07 C 57/055,** C 07 C 57/02, C 07 C 51/25, B 01 J 23/28

(21) Application number: **83303272.5**

(22) Date of filing: **07.06.83**

(54) **Catalysts, their preparation and use in vapour phase oxidation of unsaturated aldehydes.**

(30) Priority: **07.06.82 US 385365**

(43) Date of publication of application:
**14.03.84 Bulletin 84/11**

(45) Publication of the grant of the patent:
**06.08.86 Bulletin 86/32**

(84) Designated Contracting States:
**AT BE CH DE FR GB IT LI LU NL SE**

(56) References cited:
**EP-A-0 000 835**
**EP-A-0 043 100**
**DE-A-2 440 329**
**DE-A-3 010 434**
**DE-B-2 460 541**
**DE-B-2 523 757**
**FR-A-2 447 364**
**GB-A-1 478 828**

(73) Proprietor: **Rohm and Haas Company**
**Independence Mall West**
**Philadelphia, Pennsylvania 19105 (US)**

(72) Inventor: **Kennelly, William James**
**6 Churchill Lane**
**Newton Pennsylvania 18940 (US)**
Inventor: **Kirch, Lawrence Samuel**
**871 Oriole Lane**
**Huntingdon Valley Pennsylvania 19006 (US)**

(74) Representative: **Angell, David Whilton et al**
**Rohm and Haas Company Patent Department**
**Chesterfield House Bloomsbury Way**
**London WC1A 2TP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 688 B1

**Description**

This invention concerns catalysts, their preparation and use in vapour phase oxidation of unsaturated aldehydes.

By the oxidation process of the invention acrylic acid and/or methacrylic acid can be made from acrolein and/or methacrolein.

Catalysts and/or procedures known for the preparation of acrylic acid and methacrylic acid and other unsaturated acids are disclosed in the following U.S. Patents: 3,761,516; 3,795,703; 3,773,692; 3,875,220; 3,998,876; 4,000,088; 4,051,179; 4,070,397 and 4,075,244, and in DE—B—2,460,541 and DE—B—2,523,757.

For example, U.S. 4,051,179 discloses a process for preparing acrylic and/or methacrylic acid utilizing a catalyst having the empirical formula:—

$$P_aM_bAs_cX_dY_eZ_fO_g$$

wherein a is within the range of 0.03 to 0.2, b is 1, c is within the range of 0.015 to 0.15, d is within the range of 0.003 to 1, e is within the range of 0.003 to 0.417, f is within the range 0.003 to 1, g is determined by the degree of oxidation of the elements present in the catalyst, and wherein X is copper and/or vanadium, Y is at least one alkali metal element, and Z is at least one metal selected from magnesium, aluminium, silicon, calcium, titanium, zirconium, silver, antimony, tellurium, barium and tantalum. However, as discussed hereinafter, a comparison of the catalysts of the present invention with the catalyst of Example 1 of U.S. 4,051,179 (i.e. the only catalyst of the US Patent the preparation of which is described in detail) illustrates the superiority of the catalysts of the present invention.

DE—B—2,460,541 discloses a catalyst for use in the preparation of methacrylic acid, the catalyst having the empirical formula:—

$$Y_aX_bP_cMo_dV_eO_f$$

wherein Y is at least one of the metals copper, cobalt, zirconium, bismuth, antimony and arsenic, X is at least one of the alkali metals sodium, calcium, rubidium and caesium, and wherein a is 0 to 8.0, b is 0 to 3.0, c is 0.5 to 5.0, d is 12, e is 0.5 to 5.0 and f is a value determined by the valences and atomic ratios of the other constituent elements.

According to this invention there is provided catalyst represented by the formula

$$Mo_{12}P_aX^1_bV_cAs_dCu_eSb_fX^2_gO_x$$

wherein $X^1$ is one or more of the following elements: caesium, thallium and rubidium;

$X^2$ is one or more elements from the following: tungsten, niobium, tantalum, zirconium, tin, iron, zinc, cobalt, bismuth, boron, rhenium, tellurium and potassium;

a is 0.85 to 1.2,
b is 0.75 to 1.25,
c is 0.05 to 0.7,
d is 0.5 to 2.0,
e is 0 to 0.4,
f is 0 to 0.3,
g is 0 to 2.0, and
x is the number of oxygen atoms required to satisfy the valence requirements of the other elements present.

In embodiments of the invention wherein $X^1$ (and/or $X^2$) represents more than one element, then b (and/or g) represents(s) the individual number of atoms of each element represented.

Preferred catalysts of this invention are those of the formula:
wherein
a is 1 to 1.2,
b is 1 to 1.25,
c is 0.2 to 0.7,
d is 0.5 to 1.0,
e is 0 to 0.25,
f is 0 to 0.2,
g is 0 to 1.0; and
X is one or more of the following elements: bismuth, tungsten, cobalt, zinc or tin.

The most preferred catalysts are those represented by the formula:

$$Mo_{12}P_aCs_bV_cAs_dCu_eSb_fO_x$$

wherein
a is 1 to 1.2,

b is 1 to 1.25,
c is 0.2 to 0.6,
d is 0.5 to 1,
e is 0.2 to 0.25,
f is 0.1 to 0.2
x is as defined above.

Catalysts having this formula may afford the highest yield of desired product.

Of course in the case of individual catalysts a to g will be individual members wherein, as is often or usually the case, in a mixed catalyst one or more of said a to g will represent an average number in the stated range.

The catalyst of this invention may be employed in the supported or unsupported form. If a support is employed, any known support, such as alumina, pumice, silicon carbide, zirconia, silica, alumina-silica and perlite that are stable under the reaction conditions, may be employed. Alumina-silica is the preferred carrier.

The invention also provides a process for preparing a catalyst as defined above which comprises

(a) forming a suspension of molybdenum trioxide in water, adding phosphoric acid to said suspension, heating the resulting suspension to 60° to 95°C for at least sufficient time to form phosphomolybdic acid and then adding ammonium hydroxide thereto, the remaining required or allowable elements being added to the suspension in the form of oxides or salts or compounds convertible on heating into the corresponding oxides either before or after the addition of the phosphoric acid to form catalyst precursor

(b) isolating the catalyst precursor, and

(c) calcining the isolated precursor at a temperature of 350° to 450°C.

We have found that by employing particular chemicals and particular calcining techniques we may obtain catalysts which surprisingly yield conversion rates consistently in excess of 90% and selectivities consistently above 75%.

In general, a suitable process for preparing catalyst according to the invention may be as follows: adding molybdenum trioxide to water followed by the addition of phosphoric acid. This reaction mixture is heated to a temperature of 60° to 95°C for a period of time sufficient to form phosphomolybdic acid, usually at least one hour, and, preferably, three hours. The remaining desired catalyst elements are then added as their oxides or salts or compounds convertible on heating into oxides. Finally, ammonium hydroxide is added. The reaction to form the catalyst precursor, i.e. the uncalcined catalyst, may be regarded as substantially complete at this time. The water is removed conveniently by heating at a temperature of 50° to 70°C, which usually takes from 10 to 20 hours. Other methods of removing water may be employed, such as spray drying. Any remaining water is removed by well known methods such as by heating at a temperature in excess of 100°C in a vacuum oven. We have found it convenient to remove the water at a temperature of from 160° to 170°C in a vacuum oven for at least 4 hours.

Though not essential to the performance of the catalyst, for convenience in calcining and in loading the tubes of a tubular oxidation reactor, the dried, uncalcined catalyst is crushed to a convenient particle size, for example 8 to 18 mesh (ASTM Standard).

The dried catalyst is then heated to a temperature of 350° to 450°C, and preferably at a temperature of 385° to 415°C, most preferably about 400°C, wherein the calcining temperature is achieved as rapidly as possible and, preferably, in no longer than about 1 hour and more preferably in no longer than about 20 minutes.

Calcining is a thermal treatment of a catalyst precursor which causes a chemical or structural transformation of the precursor into a catalytically active form.

The most preferred catalysts are those prepared employing molybdenum trioxide. Other sources of molybdenum such as ammonium heptamolybdate may afford poor catalysts. In addition, we have found that vanadium is essential for activation and that arsenic and vanadium are necessary if the catalyst is to have a reasonable life.

We have found that potassium without cesium in the catalyst can cause a decrease in activity compared to the employment of caesium alone or caesium and potassium.

Another aspect of the present invention is the use of catalysts according to the invention in the preparation of acrylic acid and/or methacrylic acid by vapour phase oxidation of acrolein and/or methacrolein. Conveniently the appropriate aldehyde or aldehydes are comprised in an organic feed.

Accordingly in a further process aspect of the invention a mixture of organic feed containing acrolein and/or methacrolein, in vapour form, and molecular oxygen, optionally, in the presence of steam or other diluents, is contacted with a catalyst of the above composition preferably at a temperature of 290° to 320°C for a contact time sufficient to convert the feed to the corresponding acid(s). Of the two possible conversions, namely the conversion of acrolein to acrylic acid and methacrolein to methacrylic acid, the latter is the more interesting. The contact time may vary from about 2 to about 6 seconds or more. The reaction can be conducted under atmospheric, superatmospheric or subatmospheric pressures. However, in general, pressures slightly above atmospheric are preferred, such as 68.95 kPa gauge (10 psig).

Any source of oxygen may be employed in the process, but for economic reasons it is preferred that air be employed as the source of oxygen.

Diluents such as water, nitrogen, carbon dioxide and the like may be present in the reaction mixture.

In general, apparatus of the type suitable for carrying out oxidation reactions in the vapour phase may be employed in this process. The process may be conducted either continuously or intermittently. The catalyst bed may be a fixed-bed employing a large particulate or pelletized catalyst, in the alternative, a so-called "fluidized" bed of catalyst may be employed.

The products of the catalyst oxidation reaction may be recovered by any convenient method such as those known in the art. One such method involves scrubbing the effluent gases from the reactor with cold water or another appropriate solvent to remove the products of the reaction. The ultimate recovery of the product may be accompanied by conventional means, such as distillation or solvent extraction.

In order that the invention may be further illustrated, the following embodiments of the invention are presented purely by way of example.

General reaction equipment and procedure

A one litre glass resin kettle is equipped with high torque air stirrer, glass shaft and bearing with paddle 6.35 mm (1/4 inch) cut to fit. Other equipment includes a heating mantle, variable voltage heat control, 53.3 cm (21 in.) immersion thermometer, stainless steel sieves (8 and 18 mesh) and catch pans.

The molybdenum trioxide/phosphoric acid slurry is prepared as follows: charge kettle with 800 ml deionized water, set air stirrer to create vortex, and set temperature to 40°C. Add weighed molybdenum when 40°C is reached and then add phosphoric acid. Raise the temperature to 80°C and stir 3 hours. Turn off the heat and add 300 ml water to the kettle, add the chemicals sequentially to the kettle, making sure previous chemical is dissolved. Add antimony pentachloride next to last (if used). Ammonium hydroxide is always added last. (All standard molybdenumtrioxide/phosphoric acid preparations contain ammonium hydroxide). When all chemicals have been added begin heating againt to 55°C to remove the water (overnight). Next day, if the catalyst precursor is not almost completely dry, increase the temperature to 65°—75°C. When dry, place in a vacuum oven for 4 hours at 160°C. Remove the dry material to a dessicator to cool. Grind the precursor with pestle and screen, 8—18 mesh material. Finally, calcine the 8—18 mesh catalyst precursor for 6 hours at about 400°C with 1 l/min air flow through catalyst bed to afford active catalyst.

Catalyst Examples

Example 1—$Mo_{12}P_1Cs_1V_{0.2}As_1Sb_{0.1}$

To 800 ml of deionized water is added molybdenum trioxide (120.9 grams). This reaction mixture is heated to 40°C at which time is added phosphoric acid (8.07 grams). This mixture is stirred for three hours at 80°C at which time there is added water (300 ml), caesium nitrate (13.64 grams), ammonium metavanadate (1.64 grams), arsenic pentoxide (8.28 grams), antimony pentachloride (2.09 grams) and 29.2% ammonium hydroxide (12.25 grams). The reaction mixture is stirred for 16 hours at 57°C and for 2.87 hours at 80°C. The catalyst precursor is collected and dried in the vacuum oven four hours at reduced pressure (15 $mmH_g$) at 165°C to afford 150.2 grams of catalyst precursor. The material is grounded with a pestle directly on to sieves to yield 106.9 grams of 8—18 mesh material and 43 grams of fines. The catalyst precursor is calcined for 6 hours at 400°C with an air flow of 1 litre/minute to afford active catalyst.

Example 2—$Mo_{12}P_1Cs_1V_{0.2}As_1Cu_{0.2}Sb_{0.1}$

To 800 ml of water is added molybdenum trioxide (120.9 grams). The reaction mixture is warmed to 40°C and phosphoric acid (8.07 grams) is added. The reaction mixture is stirred for three hours at 80°C and then water (300 ml), caesium nitrate (13.64 grams), ammonium metavanadate (1.64), arsenic pentoxide (8.28 grams); cuprous nitrate (3.26 grams), antimony pentachloride (2.09 grams) and 29.2% ammonium hydroxide (12.25 grams) are added. The reaction mixture is stirred at 55°C for 16.75 hours and at 80°C for 4.25 hours. The catalyst precursor is collected and dried in a vacuum oven for 4 hours at 165°C at reduced pressure (10 mmHg) to yield 148 grams. The catalyst precursor is ground with a pestle to afford 93.0 grams of 8—18 mesh material and 54.9 grams of fines. Calcining of the 93 grams for 6 hours at 400°C with an air flow of 1 litre per minute yields 86.9 grams of active catalyst.

Example 3—$Mo_{12}P_{1.2}Cs_{1.25}V_{0.4}As_{0.7}Cu_{0.25}Sb_{0.2}$

To 800 ml of water is added molybdenum trioxide (120.9 grams), arsenic pentoxide (5.8 grams) and cupric nitrate (4.07 grams). The reaction mixture is heated to 40°C. There is then added phosphoric acid (9.69 grams) and the reaction mixture heated for 3 hours at 80°C at which time additional water (350 ml) and ammonium metavanadate (3.28 grams), antimony pentachloride (4.19 grams), caesium nitrate (17.05 grams) and ammonium hydroxide (12.25 grams) are added. The reaction mixture is stirred for 17 hours at 80°C. The catalyst precursor is collected and dried in a vacuum oven for 4.5 hours at 165°C at reduced pressure (30 mmHg) to yield 153.9 grams. The catalyst precursor is ground with a pestle to afford 102.5 grams of 8—18 mesh material and 50.7 grams of fines. The 102.5 grams is calcined for 6 hours at 400°C with an airflow of 1 litre per minute to afford 87.8 grams of 8—18 mesh material and 9.6 grams of fines.

Example 4—$Mo_{12}P_1Cs_1V_{0.2}As_1Cu_{0.2}Sb_{0.1}$

To 800 ml of water is added molybdenum trioxide (120.9 grams). The mixture is heated to 40°C and phosphoric acid (8.07 grams) is added. This mixture is then heated for 3 hours at 80°C. Additional water

(300 ml) and caesium nitrate (13.64 grams), ammonium metavanadate (1.64 grams); arsenic pentoxide (8.28 grams), cupric nitrate (3.26 grams), antimony pentachloride (2.09 grams) and ammonium hydroxide (12.5 grams) are added. The reaction mixture is stirred for 17.3 hours at 55°C and 2.75 hours and 80°C. The catalyst precursor is collected and dried for 4.5 hours at 165°C at reduced pressure (5 mmHg) to yield 149.1 grams of catalyst precursor. The precursor is ground to 4 mesh and calcined for 6 hours at 400°C with an airflow of 1 litre per minute to afford 140.8 grams of active catalyst.

Example 5—$Mo_{12}P_{1.2}Cs_{1.25}V_{0.2}As_1Cu_{0.2}Sb_{0.2}$

By following the general procedure and employing 120.9 grams of molybdenum trioxide, 9.69 grams phosphoric acid, 17.05 grams of caesium nitrate, 1.64 grams of ammonium metavanadate, 8.28 grams of arsenic pentoxide, 3.26 grams of copper nitrate, 4.19 grams of antimony pentachloride and 12.5 grams of ammonium hydroxide, there is obtained, after drying at 4.5 hours at 100°C at reduced pressure (5 mmHg) and for 5 hours at 165°C at reduced pressure, 135.0 grams of dried material which after grinding affords 79.2 grams of 8—18 mesh material and 55.4 grams of fines. The 8—18 mesh material is calcined for 6 hours at 400°C with an airflow of 1 litre per minute to afford 75.1 grams of active catalyst.

By employing substantially the procedures described above and by employing the following amounts of ingredients, (numbers refer to grams of material) there are obtained additional active catalysts as shown in Examples 6—9.

| Ex. No. | 6 | 7 | 8 | 9 |
|---|---|---|---|---|
| $MoO_3$ | 120.9 | 120.9 | 120.9 | 120.9 |
| $H_3PO_4$ | 9.7 | 9.7 | 12.1 | 9.7 |
| $CsNO_3$ | 17.05 | 17.05 | 20.46 | 17.05 |
| $NH_4VO_3$ | 1.64 | 4.92 | 1.64 | 4.92 |
| $As_2O_5$ | 8.28 | 4.14 | 8.28 | 5.8 |
| $SbCl_5$ | 4.19 | 4.18 | — | 4.18 |
| $NH_4OH$ (28.5%) | 12.5 | 12.5 | 12.5 | 12.5 |
| Other | $Cu(NO_3)_2$ 3.26 | — | $Cu(NO_3)_2$ 3.26 | $Cu(NO_3)_2$ 3.26 |
| | | | $Zn(NO_3)_2$ 7.26 | |
| | | | $WO_3$ 8.1 | |
| | | | $Co(NO_3)_2$ 10.2 | |

Example 6:—$Mo_{12}P_{1.2}Cs_{1.25}V_{0.2}As_1Cu_{0.2}Sb_{0.2}$

Example 7:—$Mo_{12}P_{1.2}Cs_{1.25}V_{0.6}As_{0.5}Sb_{0.2}$

Example 8:—$Mo_{12}P_{1.5}V_{0.2}As_1Cu_{0.2}W_{0.5}Co_{0.5}Zn_{0.5}$

Example 9:—$Mo_{12}P_{1.2}Cs_{1.25}V_{0.6}As_{0.7}Cu_{0.2}Sb_{0.2}$

Standard screening procedure

Pack 20.0 to 25 grams of the catalyst in a 9.53 mm (3/8") stainless steel U-tube. Reactor catalyst volume is approximately 20.0 cc. Immerse the tube in a salt bath for temperature control. Feed a mixture of air, nitrogen, water, and methacrolein over the catalyst. The water is vaporized in the air stream and the methacrolein is vaporized in the nitrogen stream. Desired conditions are as follows:

Salt bath temperature—300°C
Total flow rate—1004 mmol/hr (corresponds to 3.2 sec. space time)
Reactor pressure—68.95 kPa gauge (10.0 psig)
Air flow—479.2 mmol/hr
Nitrogen flow—183.4 mmol/hr
Methacrolein flow—40.0 mmol/hr
$H_2O$ flow—301.4 mmol/hr

Reactor effluent analyzed for acetaldehyde, acetone, acrolein, methacrolein, acetic acid, acrylic acid, and methacrylic acid by on line gas chromatography. Non-condensable gases analyzed by gas partitioning for $CO_2$, $O_2$, $N_2$, and CO. Condensed effluent is titrated for total acidity. It can also be analyzed by GLC if

necessary; particularly, for calibration purposes. A gaseous sample of effluent is automatically injected in to the online analytical system every 35 minutes, integration performed, and results calculated by computer from analytical data, flow rates, temperatures, and pressures. Averages and standard deviations for conversions, selectivities and accountabilities are computed for each run using valid samples with accountabilities of approximately 90% to 105%. The first samples of each evaluation run are omitted from this calculation.

The following tables illustrates the composition and catalytic activity of some of the catalysts of this invention. However, all catalysts embraced by the generic formula prepared by the method described in this application are expected to behave in a similar manner.

TABLE I
Catalyst compositions

| Ex. No. | Mo | Po | Cs | V | As | Cu | Sb | X |
|---|---|---|---|---|---|---|---|---|
| 1 | 12 | 1.0 | 1.0 | 0.2 | 1.0 | — | 0.1 | — |
| 2 | 12 | 1.0 | 1.0 | 0.2 | 1.0 | 0.2 | 0.1 | — |
| 3 | 12 | 1.2 | 1.25 | 0.4 | 0.7 | 0.25 | 0.2 | — |
| 4 | 12 | 1.0 | 1.0 | 0.2 | 1.0 | 0.2 | 0.1 | — |
| 5 | 12 | 1.2 | 1.25 | 0.2 | 1.0 | 0.2 | 0.2 | — |
| 6 | 12 | 1.2 | 1.25 | 0.2 | 1.0 | 0.2 | 0.2 | — |
| 7 | 12 | 1.2 | 1.25 | 0.6 | 0.5 | — | 0.2 | — |
| 8 | 12 | 1.5 | 1.25 | 0.2 | 1.0 | 0.2 | — | $W_{0.5}$ $Co_{0.5}$ $Zn_{0.5}$ |
| 9 | 12 | 1.2 | 1.25 | 0.6 | 0.7 | 0.2 | 0.2 | — |
| 10 | 12 | 1.2 | 1.25 | 0.4 | 0.25 | 0.2 | — | — |
| 11 | 12 | 1.15 | 0.75 | 0.05 | 1.7 | 0.3 | — | — |
| 12 | 12 | 1.0 | 1.0 | 0.2 | 1.0 | 0.2 | — | — |
| 13 | 12 | 1.15 | 1.25 | 0.05 | 1.3 | 0.3 | — | — |
| 14 | 12 | 1.0 | 1.0 | 0.2 | 1.6 | 0.2 | — | — |
| 15 | 12 | 1.0 | 1.0 | 0.2 | 1.5 | 0.2 | — | — |
| 16 | 12 | 1.1 | 1.12 | 0.3 | 0.85 | 0.2 | 0.15 | — |
| 17 | 12 | 1.0 | 1.0 | 0.2 | 1.4 | 0.2 | — | — |
| 18 | 12 | 1.0 | 1.0 | 0.2 | 1.0 | 0.2 | — | $Bi_{0.2}$ |
| 19 | 12 | 1.0 | 1.0 | 0.2 | 1.4 | 0.2 | 0.1 | — |
| 20 | 12 | 1.1 | 1.12 | 0.3 | 0.85 | 0.1 | 0.15 | — |
| 21 | 12 | 1.0 | 1.2 | 0.45 | 0.6 | 0.15 | — | — |
| 22 | 12 | 1.05 | 1.0 | 0.2 | 1.5 | 0.2 | — | — |
| 23 | 12 | 1.0 | 1.0 | 0.2 | 1.6 | 0.2 | — | — |
| 24 | 12 | 1.0 | 1.0 | 0.2 | 1.0 | 0.2 | 0.1 | $W_{1.0}$ |

TABLE I continued

| Ex. No. | Mo | Po | Cs | V | As | Cu | Sb | X |
|---|---|---|---|---|---|---|---|---|
| 25 | 12 | 1.0 | 1.0 | 0.2 | 1.0 | 0.2 | 0.1 | $Te_{0.1}Sn_{0.1}$ |
| 26 | 12 | 1.1 | 1.0 | 0.2 | 1.5 | 0.2 | — | — |
| 27 | 12 | 1.1 | 1.12 | 0.3 | 0.85 | 0.1 | 0.2 | — |
| 28 | 12 | 1.0 | 1.0 | 0.2 | 1.0 | 0.2 | — | $Bi_{0.1}$ |
| 29 | 12 | 1.0 | 1.0 | 0.2 | 1.0 | 0.2 | 0.1 | $Nb_{0.2}$ |
| 30 | 12 | 1.1 | 1.12 | 0.3 | 0.85 | 0.1 | 0.1 | — |
| 31 | 12 | 1.0 | 1.25 | 0.2 | 1.0 | 0.2 | 0.2 | — |
| 32 | 12 | 1.0 | 1.0 | 0.2 | 1.0 | 0.2 | 0.1 | $Te_{0.1}$ |
| 33 | 12 | 1.1 | 1.12 | 0.2 | 0.85 | 0.1 | 0.15 | — |
| 34 | 12 | 1.1 | 1.25 | 0.3 | 0.55 | 0.25 | 0.15 | — |
| 35 | 12 | 1.15 | 0.75 | 0.35 | 1.3 | 0.3 | — | — |
| 36 | 12 | 1.2 | 1.0 | 0.2 | 1.0 | 0.2 | 0.2 | — |
| 37 | 12 | 1.0 | 1.0 | 0.2 | 1.0 | 0.3 | 0.1 | — |
| 38 | 12 | 1.0 | 1.0 | 0.2 | 1.0 | 0.4 | 0.1 | — |
| 39 | 12 | 1.1 | 1.4 | 0.6 | 0.8 | 0.3 | — | — |
| 40 | 12 | 1.0 | 1.0 | 0.2 | 1.0 | 0.2 | 0.1 | $W_{0.5}$ |
| 41 | 12 | 1.1 | 1.35 | 0.3 | 0.55 | — | 0.15 | — |
| 42 | 12 | 1.0 | 1.0 | 0.2 | 1.0 | 0.2 | 0.05 | — |
| 43 | 12 | 0.85 | 1.25 | 0.05 | 1.3 | 0.3 | — | — |
| 44 | 12 | 1.2 | 1.25 | 0.6 | 0.7 | 0.2 | 0.2 | — |
| 45 | 12 | 1.0 | 1.0 | 0.2 | 1.0 | 0.2 | 0.2 | — |
| 46 | 12 | 1.0 | 1.12 | 0.3 | 0.85 | 0.1 | 0.15 | — |
| 47 | 12 | 1.2 | 1.25 | 0.2 | 1.0 | 0.2 | 0.1 | — |
| 48 | 12 | 1.0 | 1.0 | 0.2 | 1.0 | 0.2 | 0.1 | $Ta_{0.2}$ |
| 49 | 12 | 1.0 | 1.0 | 0.2 | 1.8 | 0.2 | — | — |
| 50 | 12 | 1.2 | 1.12 | 0.3 | 0.85 | 0.1 | 0.15 | — |
| 51 | 12 | 1.0 | 0.5 | 0.2 | 1.0 | 0.2 | 0.1 | $K_{0.5}$ |
| 52 | 12 | 1.0 | 1.25 | 0.4 | 0.7 | 0.2 | 0.2 | — |
| 53 | 12 | 1.1 | 1.12 | 0.4 | 0.4 | 0.85 | 0.1 | 0.15 |
| 54 | 12 | 1.0 | 0.75 | 0.2 | 1.4 | 0.2 | — | — |
| 55 | 12 | 1.0 | 1.2 | 1.0 | 0.2 | 0.1 | — | — |

TABLE I continued

| Ex. No. | Mo | Po | Cs | V | As | Cu | Sb | X |
|---|---|---|---|---|---|---|---|---|
| 56 | 12 | 1.15 | 0.75 | 0.05 | 1.3 | 0.1 | — | — |
| 57 | 12 | 1.2 | 1.25 | 0.4 | 0.7 | 0.25 | 0.05 | — |
| 58 | 12 | 1.0 | 0.5 | 0.2 | 1.4 | 0.2 | — | — |
| 59 | 12 | 1.05 | 1.25 | 0.3 | 0.55 | — | 0.15 | — |
| 60 | 12 | 0.85 | 0.75 | 0.35 | 1.3 | 0.1 | — | — |
| 61 | 12 | 0.9 | 1.0 | 0.2 | 1.5 | 0.2 | — | — |
| 62 | 12 | 1.0 | 1.0 | 0.2 | 1.0 | 0.2 | 0.1 | — |
| 63 | 12 | 1.0 | 1.25 | 0.2 | 1.4 | 0.2 | — | — |
| 64 | 12 | 1.2 | — | 0.2 | 1.0 | 0.2 | 0.2 | $Tl_{1.25}$ |
| 65 | 12 | 1.2 | 1.0 | 0.2 | 1.5 | 0.2 | — | — |
| 66 | 12 | 1.1 | 1.0 | 0.3 | 0.85 | 0.1 | 0.15 | — |
| 67 | 12 | 1.2 | 1.0 | 0.4 | 0.7 | 0.2 | 0.1 | — |
| 68 | 12 | 1.2 | 1.25 | 0.4 | 0.7 | — | 0.2 | — |
| 69 | 12 | 1.1 | 1.12 | 0.3 | 1.0 | 0.1 | 0.15 | — |
| 70 | 12 | 1.1 | 1.25 | 0.3 | 0.85 | 0.1 | 0.15 | — |
| 71 | 12 | 1.2 | 1.25 | 0.4 | 0.7 | 0.25 | — | — |
| 72 | 12 | 1.0 | 1.0 | 0.2 | 1.0 | 0.2 | 0.1 | $B_{0.3}$ |
| 73 | 12 | 1.0 | 1.0 | 0.2 | 1.0 | 0.1 | 0.1 | — |
| 74 | 12 | 1.0 | 1.0 | 0.2 | 1.0 | 0.2 | 0.1 | $W_{2.0}$ |
| 75 | 12 | 1.2 | 1.25 | 0.18 | 0.7 | — | 0.2 | — |
| 76 | 12 | 1.2 | 1.25 | 0.8 | 0.4 | 0.2 | 0.2 | — |
| 77 | 12 | 1.0 | 1.0 | 0.2 | 0.2 | 0.2 | — | — |
| 78 | 12 | 1.1 | 1.12 | 0.3 | 0.7 | 0.1 | 0.15 | — |
| 79 | 12 | 1.0 | 1.0 | 0.6 | 0.85 | 0.2 | 0.1 | — |
| 80 | 12 | 1.15 | 0.75 | 0.35 | 1.7 | 0.1 | — | — |
| 81 | 12 | 1.0 | — | 0.2 | 1.0 | 0.2 | 0.1 | $Tl_{1.0}$ |
| 82 | 12 | 1.0 | 1.0 | 0.2 | 1.0 | — | — | — |
| 83 | 12 | 1.0 | 1.0 | 0.2 | 1.2 | 0.2 | — | — |
| 84 | 12 | 1.2 | 1.25 | 0.4 | 0.7 | 0.25 | 0.1 | — |
| 85 | 12 | 1.0 | 1.0 | 0.2 | 1.0 | 0.5 | 0.1 | — |
| 86 | 12 | 1.0 | 1.0 | 0.2 | 1.1 | 0.2 | — | — |

TABLE I continued

| Ex. No. | Mo | Po | Cs | V | As | Cu | Sb | X |
|---|---|---|---|---|---|---|---|---|
| 87 | 12 | 1.0 | 1.25 | 0.2 | 0.7 | 0.2 | 0.1 | — |
| 88 | 12 | 1.15 | 1.25 | 0.35 | 1.3 | 0.1 | — | — |
| 89 | 12 | 1.0 | 1.25 | 0.4 | 1.0 | — | 0.1 | — |
| 90 | 12 | 1.0 | — | 0.2 | 1.0 | 0.2 | 0.1 | $Rb_1$ |
| 91 | 12 | 1.2 | 1.25 | 0.4 | 0.7 | 0.05 | 0.2 | — |
| 92 | 12 | 1.0 | 0.2 | 1.0 | 0.2 | 0.1 | — | — |
| 93 | 12 | 1.2 | 1.25 | 0.5 | 0.5 | — | 0.2 | — |
| 94 | 12 | 1.0 | 1.0 | 0.2 | 1.0 | 0.2 | 0.1 | $Re_{0.01}$ |
| 95 | 12 | 1.15 | 1.25 | 0.05 | 1.7 | 0.1 | — | — |
| 96 | 12 | 1.2 | 1.25 | 0.5 | 0.6 | — | 0.2 | — |
| 97 | 12 | 1.0 | 1.0 | 0.2 | 1.0 | 0.2 | 0.1 | $W_{0.3}Nb_{0.3}$ |
| 98 | 12 | 1.1 | 1.25 | 0.3 | 0.55 | — | 0.1 | — |
| 99 | 12 | 1.1 | 1.45 | 0.3 | 0.55 | — | 0.15 | — |
| 100 | 12 | 1.0 | 1.0 | 0.2 | 1.0 | 0.2 | 0.1 | $Zr_{0.2}$ |
| 101 | 12 | 1.2 | 1.25 | 0.4 | 1.0 | 0.2 | 0.1 | — |
| 102 | 12 | 1.2 | 1.0 | 0.2 | 0.7 | 0.2 | 0.2 | — |
| 103 | 12 | 1.0 | 1.0 | 0.2 | 0.7 | — | 0.2 | — |

TABLE II
Catalytic activity

| Ex. No. | Conversion (%) | Selectivity (%) | Yield of methacrylic acid (%) |
|---|---|---|---|
| 2 | 99.2 | 82.8 | 82.1 |
| 3 | 92.1 | 87.1 | 80.2 |
| 5 | 99.5 | 80.9 | 80.5 |
| 6 | 99.0 | 79.5 | 78.7 |
| 7 | 88.9 | 83.2 | 73.9 |
| 9 | 90.3 | 87.2 | 78.7 |
| 10 | 93.4 | 86.8 | 81.1 |
| 11 | 96.4 | 86.3 | 83.2 |
| 12 | 96.4 | 86.1 | 83.0 |
| 13 | 97.8 | 84.8 | 82.9 |
| 14 | 98.0 | 83.7 | 82.0 |

TABLE II continued

| Ex. No. | Conversion (%) | Selectivity (%) | Yield of methacrylic acid (%) |
|---|---|---|---|
| 15 | 98.6 | 85.2 | 84.0 |
| 16 | 94.9 | 84.7 | 80.4 |
| 17 | 95.5 | 86.6 | 82.7 |
| 18 | 98.4 | 82.8 | 81.5 |
| 19 | 96.7 | 86.4 | 83.5 |
| 20 | 97.1 | 82.1 | 79.7 |
| 21 | 99.3 | 77.9 | 77.4 |
| 22 | 96.5 | 85.7 | 82.7 |
| 23 | 93.5 | 88.5 | 82.7 |
| 24 | 98.1 | 83.7 | 82.1 |
| 25 | 98.1 | 81.4 | 79.9 |
| 26 | 99.0 | 81.7 | 80.9 |
| 27 | 91.8 | 87.1 | 80.0 |
| 28 | 94.1 | 83.9 | 78.9 |
| 29 | 98.5 | 82.3 | 81.1 |
| 30 | 96.6 | 83.4 | 80.6 |
| 31 | 91.8 | 87.0 | 79.9 |
| 32 | 96.8 | 83.5 | 80.8 |
| 33 | 99.0 | 78.2 | 77.4 |
| 34 | 99.3 | 77.1 | 76.6 |
| 35 | 87.5 | 88.7 | 77.6 |
| 36 | 97.7 | 81.7 | 79.8 |
| 37 | 97.4 | 82.2 | 80.1 |
| 38 | 94.5 | 83.1 | 78.5 |
| 39 | 96.8 | 83.3 | 80.6 |
| 40 | 99.4 | 78.4 | 77.9 |
| 41 | 96.8 | 80.1 | 77.5 |
| 42 | 99.1 | 77.5 | 76.8 |
| 43 | 97.3 | 83.3 | 81.1 |
| 44 | 90.3 | 87.2 | 78.1 |

TABLE II continued

| Ex. No. | Conversion (%) | Selectivity (%) | Yield of methacrylic acid (%) |
|---|---|---|---|
| 45 | 99.8 | 72.2 | 72.1 |
| 46 | 92.0 | 86.7 | 79.8 |
| 47 | 98.7 | 79.4 | 78.4 |
| 48 | 98.4 | 81.5 | 80.2 |
| 49 | 84.8 | 89.8 | 76.2 |
| 50 | 88.6 | 87.0 | 77.1 |
| 51 | 90.3 | 86.4 | 78.0 |
| 52 | 88.7 | 88.1 | 78.1 |
| 53 | 93.4 | 86.3 | 80.6 |
| 54 | 99.3 | 79.3 | 78.6 |
| 55 | 98.7 | 79.4 | 78.4 |
| 56 | 98.1 | 80.2 | 78.7 |
| 57 | 98.0 | 81.0 | 79.4 |
| 58 | 97.7 | 82.0 | 80.1 |
| 59 | 88.6 | 82.6 | 73.2 |
| 60 | 95.0 | 81.0 | 77.0 |
| 61 | 90.7 | 87.1 | 79.0 |
| 62 | 99.8 | 77.0 | 76.8 |
| 63 | 99.6 | 77.9 | 77.6 |
| 64 | 98.9 | 76.9 | 76.1 |
| 65 | 90.8 | 87.7 | 79.6 |
| 66 | 98.7 | 79.5 | 78.5 |
| 67 | 97.4 | 79.9 | 77.8 |
| 68 | 89.9 | 89.3 | 80.3 |
| 69 | 91.8 | 86.1 | 79.0 |
| 70 | 94.6 | 84.4 | 79.8 |
| 71 | 99.6 | 75.8 | 75.5 |
| 72 | 92.3 | 86.1 | 79.5 |
| 73 | 98.9 | 76.0 | 75.2 |
| 74 | 92.5 | 83.9 | 77.6 |

TABLE II continued

| Ex. No. | Conversion (%) | Selectivity (%) | Yield of methacrylic acid (%) |
|---|---|---|---|
| 75 | 87.1 | 82.0 | 71.4 |
| 76 | 84.0 | 88.4 | 74.3 |
| 77 | 99.5 | 76.4 | 76.0 |
| 78 | 98.6 | 78.8 | 77.7 |
| 79 | 82.6 | 89.9 | 74.3 |
| 80 | 82.4 | 89.6 | 73.8 |
| 81 | 97.9 | 80.0 | 78.3 |
| 82 | 98.3 | 79.7 | 78.3 |
| 83 | 92.3 | 81.4 | 75.1 |
| 84 | 96.4 | 79.9 | 77.0 |
| 85 | 95.9 | 80.3 | 77.0 |
| 86 | 99.4 | 75.9 | 75.4 |
| 87 | 99.9 | 74.1 | 74.0 |
| 88 | 82.8 | 89.5 | 74.1 |
| 89 | 84.6 | 84.3 | 71.3 |
| 90 | 95.5 | 78.0 | 74.5 |
| 91 | 90.1 | 86.1 | 77.6 |
| 92 | 99.7 | 72.6 | 72.4 |
| 93 | 84.4 | 84.6 | 71.4 |
| 94 | 97.8 | 78.5 | 76.8 |
| 95 | 78.1 | 91.1 | 71.1 |
| 96 | 85.2 | 84.3 | 71.8 |
| 97 | 99.7 | 74.6 | 74.4 |
| 98 | 97.0 | 73.6 | 71.4 |
| 99 | 91.7 | 80.4 | 73.7 |
| 100 | 89.3 | 82.3 | 73.5 |
| 101 | 87.5 | 86.4 | 76.6 |
| 102 | 99.7 | 69.8 | 69.6 |
| 103 | 97.6 | 74.7 | 72.9 |

Comparison of catalysts of this invention with known catalysts

Preparation of catalysts of this invention:

Five batches of catalyst were prepared by the following procedure. In a 0.45 Kg (1 lb.) resin kettle equipped with overhead air-driven stirrer, thermometer, temperature controller and heating mantle and 120.9 g of $MoO_3$ (0.840 mole) to 800 ml of deionized water. Heat to 40°C and add 8.07 g of 85% $H_3PO_4$ (0.070 mole). Heat to 80°C and hold at this temperature for 3 hours. Stop heating and replace evaporated water, then add sequentially 13.64 g of $CsNO_3$ (0.070 mole), 1.64 g of $NH_4VO_3$ (0.014 mole), 8.28 g of $As_2O_5$ (0.035 mole), 3.26 g of $Cu(NO_3)_2$ 2.5 $H_2O$ (0.014 mole), 2.09 g of $SbCl_5$ (0.007 mole), and 3 moles of concentrated $NH_4OH$ for every 12 moles of $MoO_3$ (0.021 mole). Heat at 55°C for 17 hours then increase temperature to 80°C to evaporate remaining water. Dry at 165°C for 4 hours at a pressure of 50 torr to give 148.2 g. Calcine at 400°C for 6 hours. Grind to powder—yield 139.9 g. Add 1% graphite by weight and pelletize to 3.18×3.18 mm (1/8×1/8") pellets. Catalysts of Example numbers 104, 105, 106, 107 and 108 were prepared in this manner. The composition of these catalysts is

$Mo_{12}P_1Cs_{V.2}As_1Cu_{.2}Sb_{.1}Ox$ (or in the terms of U.S. 4,051,179;

$Mo_1P_{0.083}Cs_{0.083}V_{0.017}As_{0.083}Cu_{0.017}Sb_{0.0083}O_x$).

Preparation of known catalyst:

Example 1 from U.S. 4,051,179. In the apparatus used in Examples 104 to 108 place 250 ml of deionized water and 88.5 g of ammonium heptamolybdate (0.0716 mole). Heat to 60°C and add 4.8 g of 85% $H_3PO_4$ (0.0416 mole) and 7.1 g of 50% aqueous $H_3AsO_4$ (0.025 mole), then add 2.02 g of $Cu(NO_3)_2 \cdot 3H_2O$ (0.00836 mole) in 50 ml of deionized water, 2.44 g of $NH_4VO_3$ (0.0209 mole) in 75 ml of deionized water, add 4.22 g of $KNO_3$ (0.0417 mole) and 5.37 g of $Mg(NO_3)_2 \cdot 6H_2O$ (0.0209 mole) in 50 ml of deionized water. Stir at 60°C for 20 hours. Dry at 130°C (1 atm) for 16 hours to yield 99.5 g of dry material. Grind to powder and add 2% stearic acid and 1% graphite by weight. Pelletize to 3.18×3.18 mm (1/8×1/8") and calcine in a programmed furnace increasing the temperature by 20°C per hour from 100° to 400°C and holding at 400°C for 5 hours. Five catalysts were made by this procedure and designated K-1, K-2, K-3, K-4 and K-5. The composition of these catalysts is

$Mo_1P_{0.083}As_{0.05}V_{0.042}K_{0.083}Mg_{0.042}O_x$.

Evaluation:

Catalysts were evaluated by placing about 22.5 g of catalyst in a 9.53 mm (3/8") stainless steel tube and passing a feed with a $O_2$:$N_2$:$H_2O$:Methacrolein ratio of 10:65:20:5 over the catalyst with a space time of about 3.6 sec. The feed was at a pressure of 55.16 kPa gauge (8 psig). The reactor was held at 290°C with a molten salt bath. Samples of effluent were periodically evaluated by an on-line gas chromatograph and an on-line gas partitioner. One obvious physical difference between catalysts of this invention and those made according to the patent procedure is that the catalysts of this invention are about 30% denser than the other catalysts.

Results:

The average conversion, selectivity, and yield of each catalyst is given in Table III. The mean results and their standard deviations were also calculated for each of the preparations and are presented in Table IV. A test of statistical significance at the 95% confidence level indicates that the catalysts of this invention have a significantly higher conversion and yield than the patent example catalysts.

Conclusion:

The data show that the catalysts of this invention are superior to the known catalysts of example 1 of U.S. 4,051,179.

TABLE III

| Example | Conv | Sel | Yield |
|---|---|---|---|
| 104 | 99.1 | 75.2 | 74.5 |
| 105 | 99.4 | 69.6 | 69.2 |
| 106 | 99.3 | 72.6 | 72.1 |
| 107 | 99.2 | 68.4 | 67.9 |
| 108 | 99.3 | 74.7 | 74.2 |
| K-1 | 63.2 | 81.8 | 51.7 |
| K-2 | 60.6 | 80.6 | 48.8 |
| K-3 | 51.5 | 83.1 | 42.8 |
| K-4 | 22.7 | 84.1 | 19.1 |
| K-5 | 64.4 | 82.1 | 52.9 |

TABLE IV

| Variable | N | Mean | Standard deviation |
|---|---|---|---|
| | Catalysts of exs. 104—108 | | |
| Conv | 5 | 99.26 | 0.11401754 |
| Sel | 5 | 72.10 | 3.02324329 |
| Yield | 5 | 71.58 | 2.95245660 |
| | Known catalysts | | |
| Conv | 5 | 52.48 | 17.39790217 |
| Sel | 5 | 82.34 | 1.32778010 |
| Yield | 5 | 43.06 | 13.95145154 |

**Claims for the Contracting States: BE, CH, DE, FR, GB, IT, LI, LU, NL, SE**

1. Catalyst represented by the formula

$$Mo_{12}P_aX^1_bV_cAs_dCu_eSb_fX^2_gO_x$$

wherein $X^1$ is one or more of the following elements: caesium, thallium and rubidium;

$X^2$ is one or more elements from the following: tungsten, niobium, tantalum, zirconium, tin, iron, zinc, cobalt, bismuth, boron, rhenium, tellurium, and potassium;

a is 0.85 to 1.2,
b is 0.75 to 1.25,
c is 0.05 to 0.7,
d is 0.5 to 2.0,
e is 0 to 0.4,
f is 0 to 0.3,
g is 0 to 2.0, and
x is the number of oxygen atoms required to satisfy the valence requirements of the other elements present.

2. Catalyst according to claim 1 represented by the formula:

$$Mo_{12}P_aCs_bV_cAs_dCu_eSb_fX^2_gO_x$$

wherein
a is 1 to 1.2,
b is 1 to 1.25,
c is 0.2 to 0.7,
d is 0.5 to 1.0,
e is 0 to 0.25,
f is 0 to 0.2,
g is 0 to 1.0,
x is the number of oxygen atoms required to satisfy the valence requirements of the other elements present, and
$X^2$ is one or more of the following elements: bismuth, tungsten, cobalt, zinc and tin.

3. Catalyst according to either preceding claim represented by the formula:

$$Mo_{12}P_aCs_bV_cAs_dCu_eSb_fO_x$$

wherein
a is 1 to 1.2,
b is 1 to 1.25,
c is 0.2 to 0.6
d is 0.5 to 1,
e is 0.1 to 0.2,
f is 0.1 to 0.2, and
x is the number of oxygen atoms required to satisfy the valence requirements of the other elements present.

4. Catalyst according to any preceding claim containing one or more of the following individual catalysts:

$Mo_{12}P_{1.15}Cs_{0.75}V_{0.05}As_{1.7}Cu_{0.3}O_x$,

$Mo_{12}P_1Cs_1V_{0.2}As_1Cu_{0.2}Sb_{0.1}O_x$,

$Mo_{12}P_{1.0}Cs_{1.0}V_{0.2}As_{1.0}Cu_{0.2}O_x$

$Mo_{12}P_{1.0}Cs_{1.0}V_{0.2}As_{1.5}Cu_{0.2}O_x$,

$Mo_{12}P_{1.15}Cs_{1.25}V_{0.05}As_{1.3}Cu_{0.3}O_x$,

$Mo_{12}P_{1.0}Cs_{1.0}V_{0.2}As_{1.6}Cu_{0.2}O_x$,

$Mo_{12}P_{1.1}Cs_{1.12}V_{0.3}As_{0.85}Cu_{0.2}Sb_{0.15}O_x$,

$Mo_{12}P_{1.0}Cs_{1.0}V_{0.2}As_{1.4}Cu_{0.2}O_x$,

$Mo_{12}P_{1.2}Cs_{1.25}V_{0.4}As_{0.7}Sb_{0.2}O_x$,

$Mo_{12}P_{1.2}Cs_{1.25}V_{0.2}As_{1.0}Cu_{0.2}Sb_{0.2}O_x$,

$Mo_{12}P_{1.15}Cs_{0.75}V_{0.35}As_{1.3}Cu_{0.3}O_x$,

$Mo_{12}P_{1.2}Cs_{1.25}V_{0.6}As_{0.5}Sb_{0.2}O_x$, and

$Mo_{12}P_{1.2}Cs_{1.25}V_{0.6}As_{0.7}Cu_{0.2}Sb_{0.2}O_x$.

5. A process for preparing catalyst according to any preceding claim which comprises (a) forming a suspension of molybdenum trioxide in water, adding phosphoric acid to said suspension, heating the resulting suspension to 60° to 95°C for at least sufficient time to form phosphomolybdic acid and then adding ammonium hydroxide thereto, the remaining required or allowable elements being added to the suspension in the form of oxides or salts or compounds convertible on heating into the corresponding oxides either before or after the addition of the phosphoric acid to form catalyst precursor.
(b) isolating the catalyst precursor, and
(c) calcining the isolated precursor at a temperature of 350° to 450°C.

6. A process as claimed in Claim 5 wherein the suspension formed in step (a) is heated for at least 1 hour preferably at least 3 hours.

7. A process according to Claim 5 or 6 wherein said precursor is calcined at a temperature of 385° to 415°C, preferably about 400°C.

8. A process for the vapour phase oxidation of acrolein and/or methacrolein to acrylic and/or methacrylic acid which comprises oxidizing said acrolein and/or methacrolein in the presence of molecular oxygen in the presence of catalyst as claimed in any of claims 1 to 4.

9. A process as claimed in Claim 8 wherein the oxidation is carried out at a temperature of 290° to 320°C.

10. A process as claimed in Claim 8 or 9 wherein the catalyst has been prepared by a process as claimed in any one of claims 5 to 7.

**Claims for the Contracting State: AT**

1. A process for vapour phase oxidation of acrolein and/or methacrolein to acrylic acid and/or methacrylic acid which comprises oxidizing said acrolein and/or methacrolein in the presence of molecular oxygen by passing the reaction mixture over catalyst represented by the formula:

$$Mo_{12}P_aX^1_bV_cAs_dCu_eSb_fX^2_gO_x$$

wherein $X^1$ is one or more of the following elements: caesium, thallium and rubidium;

$X^2$ is one or more elements from the following: tungsten, niobium, tantalum, zirconium, tin, iron, zinc, cobalt, bismuth, boron, rhenium, tellurium and potassium;

a is 0.85 to 1.2,
b is 0.75 to 1.25,
c is 0.05 to 0.7,
d is 0.5 to 2.0,
e is 0 to 0.4,
f is 0 to 0.3,
g is 0 to 2.0, and
x is the number of oxygen atoms required to satisfy the valence requirements of the other elements present.

2. A process as claimed in Claim 1 wherein the oxidation is carried out at a temperature of 290°C to 320°C.

3. A process as claimed in either preceding claim wherein the catalyst is represented by the formula:

$$Mo_{12}P_aCs_bV_cAs_dCu_eSb_fX^2_gO_x$$

wherein
a is 1 to 1.2,
b is 1 to 1.25,
c is 0.2 to 0.7,
d is 0.5 to 1.0,
e is 0 to 0.25,
f is 0 to 0.2,
g is 0 to 1.0
x is the number of oxygen atoms required to satisfy the valence requirements of the other elements present, and
$X^2$ is one or more of the following elements: bismuth, tungsten, cobalt, zinc and tin.

4. A process as claimed in any preceding claim wherein the catalyst is represented by the formula:

$$Mo_{12}P_aCs_bV_cAs_dCu_eSb_fO_x$$

wherein
a is 1 to 1.2,
b is 1 to 1.25,
c is 0.2 to 0.6,
d is 0.5 to 1,
e is 0.1 to 0.2
f is 0.1 to 0.2, and
x is the number of oxygen atoms required to satisfy the valence requirements of the other elements present.

5. A process as claimed in any preceding claim wherein the catalyst contains one or more of the following individual catalysts:

$Mo_{12}P_{1.15}Cs_{0.75}V_{0.05}As_{1.7}Cu_{0.3}O_x$,

$Mo_{12}P_1Cs_1V_{0.2}As_1Cu_{0.2}Sb_{0.1}O_x$,

$Mo_{12}P_{1.0}Cs_{1.0}V_{0.2}As_{1.0}Cu_{0.2}O_x$,

$Mo_{12}P_{1.0}Cs_{1.0}V_{0.2}As_{1.5}Cu_{0.2}O_x$,

$Mo_{12}P_{1.15}Cs_{1.25}V_{0.05}As_{1.3}Cu_{0.3}O_x$,

$Mo_{12}P_{1.0}Cs_{1.0}V_{0.2}As_{1.6}Cu_{0.2}O_x$,

$Mo_{12}P_{1.1}Cs_{1.12}V_{0.3}As_{0.85}Cu_{0.2}Sb_{0.15}O_x$,

$Mo_{12}P_{1.0}Cs_{1.0}V_{0.2}As_{1.4}Cu_{0.2}O_x$,

$Mo_{12}P_{1.2}Cs_{1.25}V_{0.4}As_{0.7}Sb_{0.2}O_x$,

$Mo_{12}P_{1.2}Cs_{1.25}V_{0.2}As_{1.0}Cu_{0.2}Sb_{0.2}O_x$,

$Mo_{12}P_{1.15}Cs_{0.75}V_{0.35}As_{1.3}Cu_{0.3}O_x$,

$Mo_{12}P_{1.2}Cs_{1.25}V_{0.6}As_{0.5}Sb_{0.2}O_x$, and

$Mo_{12}P_{1.2}Cs_{1.25}V_{0.6}As_{0.7}Cu_{0.2}Sb_{0.2}O_x$.

6. A catalyst composition comprising catalyst as defined in any preceding claim in supported form.

7. A composition as claimed in claim 6 wherein the support is one of the following: alumina, pumice, silicon carbide, zirconia, silica, alumina-silica and perlite, preferably alumina-silica.

8. A method of making catalyst useful either in a process as claimed in any one of claims 1 to 5 or in forming a catalyst composition as claimed in claim 6 or 7, which method comprises (a) forming a suspension of molybdenum trioxide in water, adding phosphoric acid to said suspension, heating the resulting suspension to 60° to 95°C for at least sufficient time to form phosphomolybdic acid and then adding ammonium hydroxide thereto, the remaining required or allowable elements being added to the suspension in the form of oxides or salts or compounds convertible on heating into the corresponding oxides either before or after the addition of the phosphoric acid to form catalyst precursor

(b) isolating the catalyst precursor, and

(c) calcining the isolated precursor at a temperature of 350° to 450°C.

9. A method as claimed in claim 8 wherein the suspension formed in step (a) is heated for at least 1 hour preferably at least 3 hours.

10. A method as claimed in Claim 8 or 9 wherein said precursor is calcined at a temperature of 385° to 415°C preferably about 400°C.

11. A process as claimed in any one of claims 1 to 5 wherein the catalyst is a catalyst composition as claimed in claim 6 or 7 or catalyst made by a method as claimed in claim 8 or 9.

**Patentansprüche: BE, CH, DE, FR, GB, IT, LI, LU, NL, SE**

1. Katalysator der Formel

$$Mo_{12}P_aX^1_bV_cAs_dCu_eSb_fX^2_gO_x$$

worin $X^1$ eines oder mehrere der folgenden Elemente ist: Cäsium, Thallium und Rubidium;

$X^2$ eines oder mehrere der folgenden Elemente ist: Wolfram, Niob, Tanatal, Zirkonium, Zinn, Eisen, Zink, Kobalt, Wismut, Bor, Rhenium, Tellur und Kalum;

a 0,85 bis 1,2 ist,
b 0,75 bis 1,25 ist,
c 0,05 bis 0,7 ist,
d 0,5 bis 2,0 ist,
e 0 bis 0,4 ist,
f 0 bis 0,3 ist,
g 0 bis 2,0 ist, und
x die Anzahl der Sauerstoffatome ist, die erforderlich sind, um die Valzenbedürfnisse der anderen vorliegenden Elemente zu befriedigen.

2. Katalysator nach Anspruch 1 der Formel:

$$Mo_{12}P_aCs_bV_cAs_dCu_eSb_fX^2{}_gO_x$$

worin

a 1 bis 1,2 ist,

b 1 bis 1,25 ist,
c 0,2 bis 0,7 ist,
d 0,5 bis 1,0 ist,
e 0 bis 0,25 ist,
f 0 bis 0,2 ist,
g 0 bis 1,0 ist,
x die Anzahl der Sauerstoffatome ist, die erforderlich sind, um die Valenzbedürfnisse der anderen vorliegenden Elemente zu befriedigen, und
$X^2$ ein oder mehreres oder folgenden Elemente ist: Wismut, Wolfram, Kobalt, Zink und Zinn.

3. Katalysator nach einem der vorhergehenden Ansprüche der Formel

$$Mo_{12}P_aCs_bV_cAs_dCu_eSb_fO_x$$

worin
a 1 bis 1,2 ist,
b 1 bis 1,25 ist,
c 0,2 bis 0,6 ist,
d 0,5 bis 1 ist,
e 0,1 bis 0,2 ist,
f 0,1 bis 0,2 ist, und
x die Anzahl der Sauerstoffatome ist, die erforderlich sind, um die Valenzbedürfnisse der anderen vorliegenden Elemente zu befriedigen.

4. Katalysator nach einem der vorhergehenden Ansprüche, der einen oder mehrere der folgenden individuellen Katalysatoren enthält:

$Mo_{12}P_{1.15}Cs_{0.75}V_{0.05}As_{1.7}Cu_{0.3}O_x$,

$Mo_{12}P_1Cs_1V_{0.2}As_1Cu_{0.2}Sb_{0.1}O_x$,

$Mo_{12}P_{1.0}Cs_{1.0}V_{0.2}As_{1.0}Cu_{0.2}O_x$

$Mo_{12}P_{1.0}Cs_{1.0}V_{0.2}As_{1.5}Cu_{0.2}O_x$,

$Mo_{12}P_{1.15}Cs_{1.25}V_{0.05}As_{1.3}Cu_{0.3}O_x$,

$Mo_{12}P_{1.0}Cs_{1.0}V_{0.2}As_{1.6}Cu_{0.2}O_x$,

$Mo_{12}P_{1.1}Cs_{1.12}V_{0.3}As_{0.85}Cu_{0.2}Sb_{0.15}O_x$,

$Mo_{12}P_{1.0}Cs_{1.0}V_{0.2}As_{1.4}Cu_{0.2}O_x$,

$Mo_{12}P_{1.2}Cs_{1.25}V_{0.4}As_{0.7}Sb_{0.2}O_x$,

$Mo_{12}P_{1.2}Cs_{1.25}V_{0.2}As_{1.0}Cu_{0.2}Sb_{0.2}O_x$,

$Mo_{12}P_{1.15}Cs_{0.75}V_{0.35}As_{1.3}Cu_{0.3}O_x$,

$Mo_{12}P_{1.2}Cs_{1.25}V_{0.6}As_{0.5}Sb_{0.2}O_x$, and

$Mo_{12}P_{1.2}Cs_{1.25}V_{0.6}As_{0.7}Cu_{0.2}Sb_{0.2}O_x$.

5. Verfahren zur Herstellung eines Katalysators gemäß einem der vorhergehenden Ansprüche, dadurch gekennzeichnet, daß
(a) eine Suspension von Molybdäntrioxid in Wasser gebildet wird, Phosphorsäure der Suspension zugesetzt wird, die erhaltene Suspension auf 60 bis 95°C während wenigstens einer Zeitspanne erhitzt wird, die dazu ausreicht, Phosphormolybdänsäure zu bilden, und dann Ammoniumhydroxid zugesetzt wird, wobei die restlichen erforderlichen oder zulässigen Elemente der Suspension in Form von Oxiden oder Salzen oder Verbindungen, die beim Erhitzen in die entsprechenden Oxide umwandelbar sind, entweder vor oder nach der Zugabe der Phosphorsäure unter Bildung des Katalysatorvorläufers zugegeben werden,
(b) der Katalysatorvorläufer isoliert wird und
(c) der isolierte Vorläufer bei einer Temperatur von 350 bis 450°C kalziniert wird.

6. Verfahren nach Anspruch 5, dadurch gekennzeichnet, daß die in der Stufe (a) gebildete Suspension während wenigstens 1 Stunde, vorzugsweise wenigstens 3 Stunden, erhitzt wird.

7. Verfahren nach Anspruch 5 oder 6, dadurch gekennzeichnet, daß der Vorläufer bei einer Temperatur von 385 bis 415°C, vorzugsweise ungefähr 400°C, kalziniert wird.

8. Verfahren zur Dampfphasenoxidation von Acrolein und/oder Methacrolein zu Acrylsäure und/oder Methacrylsäure, dadurch gekennzeichnet, daß Acrolein und/oder Methacrolein in Gegenwart von molekularem Sauerstoff in Gegenwart eines Katalysators gemäß einem der Ansprüche 1 bis 4 oxidiert wird.

9. Verfahren nach Anspruch 8, dadurch gekennzeichnet, daß die Oxidation bei einer Temperatur von 290 bis 320°C durchgeführt wird.

10. Verfahren nach einem der Ansprüche 8 oder 9, dadurch gekennzeichnet, daß der Katalysator nach einem Verfahren gemäß einem der Ansprüche 5 bis 7 hergestellt worden ist.

**Patentansprüche: AT**

1. Verfahren zur Dampfphasenoxidation von Acrolein und/oder Methacrolein zu Acrylsäure und/oder Methacrylsäure, dadurch gekennzeichnet, daß Acrolein und/oder Methacrolein in Gegenwart von molekularem Sauerstoff oxidiert werden durch Überleiten der Reaktionsmischung über einen Katalysator der Formel:

$$Mo_{12}P_aX^1{}_bV_cAs_dCu_eSb_fX^2{}_gO_x$$

worin $X^1$ eines oder mehrere der folgenden Elemente ist: Cäsium, Thallium und Rubidium;

$X^2$ eines oder mehrer der folgenden Elemente ist: Wolfram, Niob, Tantal, Zirkonium, Zinn, Eisen, Zink, Kobalt, Wismut, Bor, Rhenium, Tellur und Kalium;

a 0,85 bis 1,2 ist,
b 0,75 bis 1,25 ist,
c 0,05 bis 0,7 ist,
d 0,5 bis 2,0 ist,
e 0 bis 0,4 ist,
f 0 bis 0,3 ist,
g 0 bis 2,0 ist, und
x die Anzahl der Sauerstoffatome ist, die erforderlich ist, um die Valenzbedürfnisse der anderen vorliegenden Elemente zu befriedigen.

2. Verfahren nach Anspruch 1, dadurch gekennzeichnet, daß die Oxidation bei einer Temperatur von 290 bis 320°C durchgeführt wird.

3. Verfahren nach einem der vorhergehenden Ansprüche, dadurch gekennzeichnet, daß der Katalysator der Formel

$$Mo_{12}P_aCs_bV_cAs_dCu_eSb_fX^2_gO_x$$

entspricht, worin
a 1 bis 1,2 ist,
b 1 bis 1,25 ist,
c 0,2 bis 0,7 ist,
d 0,5 bis 1,0 ist,
e 0 bis 0,25 ist,
f 0 bis 0,25 ist,
g 0 bis 1,0 ist
x die Anzahl der Sauerstoffatome ist, die erforderlich sind, um die Valenzanforderungen der anderen vorliegenden Elemente zu beriedigen, und
$X^2$ eines oder mehrere der folgenden Elemente ist: Wismut, Wolfram, Kobalt, Zink und Zinn.

4. Verfahren nach einem der vorhergehenden Ansprüche, dadurch gekennzeichnet, daß der Katalysator der Formel

$$Mo_{12}P_aCs_bV_cAs_dCu_eSb_fO_x$$

entspricht, worin
a 1 bis 1,2 ist,
b 1 bis 1,25 ist,
c 0,2 bis 0,6 ist,
d 0,5 bis 1 ist,.
e 0,1 bis 0,2 ist,
f 0,1 bis 0,2 ist, und
x die Anzahl der Sauerstoffatome ist, die erforderlich sind, um die Wertigkeitsanforderungen der anderen vorliegenden Elemente zu befriedigen.

5. Verfahren nach einem der vorhergehenden Ansprüche, dadurch gekennzeichnet, daß der Katalysator einen oder mehreren der folgenden individuellen Katalysatoren enthält:

$Mo_{12}P_{1.15}Cs_{0.75}V_{0.05}As_{1.7}Cu_{0.3}O_x$,

$Mo_{12}P_1Cs_1V_{0.2}As_1Cu_{0.2}Sb_{0.1}O_x$,

$Mo_{12}P_{1.0}Cs_{1.0}V_{0.2}As_{1.0}Cu_{0.2}O_x$,

$Mo_{12}P_{1.0}Cs_{1.0}V_{0.2}As_{1.5}Cu_{0.2}O_x$,

$Mo_{12}P_{1.15}Cs_{1.25}V_{0.05}As_{1.3}Cu_{0.3}O_x$,

$Mo_{12}P_{1.0}Cs_{1.0}V_{0.2}As_{1.6}Cu_{0.2}O_x$,

$Mo_{12}P_{1.1}Cs_{1.12}V_{0.3}As_{0.85}Cu_{0.2}Sb_{0.15}O_x$,

$Mo_{12}P_{1.0}Cs_{1.0}V_{0.2}As_{1.4}Cu_{0.2}O_x$,

$Mo_{12}P_{1.2}Cs_{1.25}V_{0.4}As_{0.7}Sb_{0.2}O_x$,

$Mo_{12}P_{1.2}Cs_{1.25}V_{0.2}As_{1.0}Cu_{0.2}Sb_{0.2}O_x$,

$Mo_{12}P_{1.15}Cs_{0.75}V_{0.35}As_{1.3}Cu_{0.3}O_x$,

$Mo_{12}P_{1.2}Cs_{1.25}V_{0.6}As_{0.5}Sb_{0.2}O_x$, and

$Mo_{12}P_{1.2}Cs_{1.25}V_{0.6}As_{0.7}Cu_{0.2}Sb_{0.2}O_x$.

6. Katalysatorzubereitung aus einem Katalysator gemäß einem der vorhergehenden Ansprüche in auf einem Träger abgeschiedener Form.

7. Zubereitung nach Anspruch 6, dadurch gekennzeichnet, daß der Träger einer der folgenden ist: Aluminiumoxid, Bimsstein, Siliziumkarbid, Zirkonoxid, Siliziumdioxid, Aluminiumoxid/Siliziumdioxid und Perlit, vorzugsweise Aluminiumoxid/Siliziumdioxid.

8. Verfahren zur Herstellung eines Katalysators, der in einem Verfahren gemäß einem der Ansprüche 1 bis 5 oder zur Herstellung einer Katalysatorzubereitung gemäß Anspruch 6 oder 7 geeignet ist, dadurch gekennzeichnet, daß

(a) eine Suspension von Molybdäntrioxid in Wasser gebildet wird, Phosphorsäure der Suspension zugesetzt wird, die erhaltene Suspension auf 60 bis 95°C während wenigstens einer Zeitspanne erhitzt wird, die dazu aus reicht, Phosphormolybdänsäure zu bilden, worauf Ammoniumhydroxid zugesetzt wird, wobei die restlichen erforderlichen oder zulässigen Elemente der Suspension in Form von Oxiden oder Salzen oder Verbindungen, die beim Erhitzen in die entsprechende Oxide umwandelbar sind, entweder vor oder nach der Zugabe der Phosphorsäure unter Bildung eines Katalysatorvorläufers zugegeben werden,

(b) der Katalysatorvorläufer isoliert wird, und

(c) der isolierte Vorläufer bei einer Temperatur von 350 bis 450°C kalziniert wird.

9. Verfahren nach Anspruch 8, dadurch gekennzeichnet, daß die in der Stufe (a) gebildete Suspension während wenigstens 1 Stunde, vorzugsweise wenigstens 3 Stundden, erhitzt wird.

10. Verfahren nach Anspruch 8 oder 9, dadurch gekennzeichnet, daß der Vorläufer bei einer Temperatur von 385 bis 415°C, vorzugsweise ungefähr 400°C, kalziniert wird.

11. Verfahren nach einem der Ansprüche 1 bis 5, dadurch gekennzeichnet, daß der Katalysator eine Katalysatorzubereitung gemäß Anspruch 6 oder 7 oder ein Katalysator ist, der nach einer Methode gemäß Anspruch 8 oder 9 hergestellt worden ist.

**Revendications Pour les états Contractants: BE, CH, DE, FR, GB, IT, LI, LU, NL, SE.**

1. Catalyseur représenté par la formule

$$Mo_{12}P_aX^1_bV_cAs_dCu_eSb_fX^2_gO_x$$

dans laquelle $X^1$ est un ou plusieurs des éléments suivants: césium, thallium et rubidium;

$X^2$ est un ou plusieurs des éléments suivants: tungstène, niobium, tantale, zirconium, étain, fer, zinc, cobalt, bismuth, bore, rhénium, tellure et potassium;

a est 0,85 à 1,2
b est 0,75 à 1,25
c est 0,05 à 0,7
d est 0,5 à 2,0
e est 0 à 0,4
f est 0 à 0,3
g est 0 à 2,0, et

x est le nombre des atomes d'oxygène nécessaires pour satisfaire aux exigences valentielles des autres éléments présents.

2. Catalyseur selon la revendication 1 représenté par la formule:

$$Mo_{12}P_aCs_bV_cAs_dCu_eSb_fX^2_gO_x$$

dans laquelle

a est 1 à 1,2
b est 1 à 1,25
c est 0,2 à 0,7
d est 0,5 à 1,0
e est 0 à 0,25
f est 0 à 0,2
g est 0,1 à 1,0
x est le nombre des atomes d'oxygène nécessaires pour satisfaire aux exigences valentielles des autres éléments présents et
$X^2$ est un ou plusieurs des éléments suivants: bismuth, tungstène, cobalt, zinc et étain.

3. Catalyseur selon l'une ou l'autre des revendications précédentes représenté par la formule:

$$Mo_{12}P_aCs_bV_cAs_dCu_eSb_fO_x$$

dans laquelle

a est 1 à 1,2
b est 1 à 1,25
c est 0,2 à 0,6
d est 0,5 à 1
e est 0,1 à 0,2
f est 0,1 à 0,2, et
x est le nombre d'atomes d'oxygène nécessaires pour satisfaire aux exigences valentielles des autres éléments présents.

4. Catalyseur selon l'une quelconque des revendications précédentes contenant un ou plusieurs des catalyseurs individuels suivants:

$Mo_{12}P_{1,15}Cs_{0,75}V_{0,05}As_{1,7}Cu_{0,3}O_x$,

$Mo_{12}P_1Cs_1V_{0,2}As_1Cu_{0,2}Sb_{0,1}O_x$,

$Mo_{12}P_{1,0}Cs_{1,0}V_{0,2}As_{1,0}Cu_{0,2}O_x$

$Mo_{12}P_{1,0}Cs_{1,0}V_{0,2}As_{1,5}Cu_{0,2}O_x$,

$Mo_{12}P_{1,15}Cs_{1,25}V_{0,05}As_{1,3}Cu_{0,3}O_x$,

$Mo_{12}P_{1,0}Cs_{1,0}V_{0,2}As_{1,6}Cu_{0,2}O_x$,

$Mo_{12}P_{1,1}Cs_{1,12}V_{0,3}As_{0,85}Cu_{0,2}Sb_{0,15}O_x$,

$Mo_{12}P_{1,0}Cs_{1,0}V_{0,2}As_{1,4}Cu_{0,2}O_x$,

$Mo_{12}P_{1,2}Cs_{1,25}V_{0,4}As_{0,7}Sb_{0,2}O_x$,

$Mo_{12}P_{1,2}Cs_{1,25}V_{0,2}As_{1,0}Cu_{0,2}Sb_{0,2}O_x$,

$Mo_{12}P_{1,15}Cs_{0.75}V_{0,35}As_{1,3}Cu_{0,3}O_x$,

$Mo_{12}P_{1,2}Cs_{1,25}V_{0,6}As_{0,5}Sb_{0,2}O_x$, et

$Mo_{12}P_{1,2}Cs_{1,25}V_{0,6}As_{0,7}Cu_{0,2}Sb_{0,2}O_x$.

5. Un procédé pour la préparation d'un catalyseur selon l'une quelconque des revendications précédentes qui comprend

(a) la formation d'une suspension de trioxyde de molybdène dans l'eau, l'addition d'acide phosphorique à ladite suspension, le chauffage de la suspension obtenue entre 60° et 95°C pendant une durée au moins suffisante pour former de l'acide phosphomolybdique, puis l'addition d'hydroxyde d'ammonium, le reste des éléments nécessaires ou acceptables étant ajouté à la suspension sous forme

d'oxydes ou de sels ou de composés convertibles par chauffage en les oxydes correspondants soit avant, soit après l'addition de l'acide phosphorique pour former un précurseur de catalyseur,

(b) l'isolement du précurseur de catalyseur, et

(c) la calcination du précurseur isolé à une température de 350° à 450°C.

6. Un procédé comme revendiqué dans la revendication 5 dans lequel la suspension formée dans le stade (a) est chauffée pendant au moins 1 heure, de préférence au moins 3 heures.

7. Un procédé selon la revendication 5 ou 6 dans lequel ledit précurseur est calciné à une température de 385° à 415°C, de préférence d'environ 400°C.

8. Un procédé pour l'oxydation en phase vapeur de l'acroléine et/ou de la méthacroléine en acide acrylique et/ou méthacrylique qui comprend l'oxydation de ladite acroléine et/ou méthacroléine en présence d'oxygène moléculaire en présence d'un catalyseur comme revendiqué dans l'une quelconque des revendications 1 à 4.

9. Un procédé comme revendiqué dans la revendication 8 dans lequel l'oxydation est effectuée à une température de 290° à 320°C.

10. Un procédé comme revendiqué dans la revendication 8 ou 9 dans lequel le catalyseur a été préparé selon un procédé comme revendiqué dans l'une quelconque des revendications 5 à 7.

**Revendications Pour l'état Contractant: AT**

1. Un procédé pour l'oxydation en phase vapeur de l'acroléine et/ou de la méthacroléine en acide acrylique et/ou acide méthacrylique qui comprend l'oxydation de ladite acroléine et/ou méthacroléine en présence d'oxygène moléculaire par passage du mélange réactionnel sur un catalyseur représenté par la formule:

$$Mo_{12}P_aX^1_bV_cAs_dCu_eSb_fX^2_gO_x$$

dans laquelle $X^1$ est un ou plusieurs des éléments suivants: césium, thallium et rubidium;

$X^2$ est un ou plusieurs des éléments suivants: tungstène, niobium, tantale, zirconium, étain, fer, zinc, cobalt, bismuth, bore, rhénium, tellure et potassium;

a est 0,85 à 1,2
b est 0,75 à 1,25
c est 0,05 à 0,7
d est 0,5 à 2,0
e est 0 à 0,4
f est 0 à 0,3
g est 0 à 2,0, et
x est le nombre d'atomes d'oxygène nécessaires pour satisfaire aux exigences valentielles des autres éléments présents.

2. Un procédé comme revendiqué dans la revendication 1 dans lequel l'oxydation est effectuée à une température de 290°C à 320°C.

3. Un procédé comme revendiqué dans l'une ou l'autre des revendications précédentes dans lequel le catalyseur est représenté par la formule:

$$Mo_{12}P_aCs_bV_cAs_dCu_eSb_fX^2_gO_x$$

dans laquelle
a est 1 à 1,2
b est 0,2 à 0,7
c est est 0,2 à 0,7
d est 0,5 à 1,0
e est 0 à 0,25
f est 0 à 0,2
g est 0 à 1,0
x est le nombre d'atomes d'oxygène nécessaires pour satisfaire aux exigences valentielles des autres éléments présents, et
$X^2$ est un ou plusieurs des éléments suivants: bismuth, tungstène, cobalt, zinc et étain.

4. Un procédé comme revendiqué dans l'une quelconque des revendications précédentes dans lequel le catalyseur est représenté par la formule

$$Mo_{12}P_aCs_bV_cAs_dCu_eSb_fO_x$$

dans laquelle
a est 1 à 1,2
b est 1 à 1,25
c est 0,2 à 0,6

d est 0,5 à 1
e est 0,1 à 0,2
f est 0,1 à 0,2, et
x est le nombre d'atomes d'oxygène nécessaires pour satisfaire aux exigences valentielles des autres éléments présents.

5. Un procédé comme revendiqué dans l'une quelconque des revendications précédentes dans lequel le catalyseur contient un ou plusieurs des catalyseurs individuels suivants:

$Mo_{12}P_{1,15}Cs_{0,75}V_{0,05}As_{1,7}Cu_{0,3}O_x$,

$Mo_{12}P_1Cs_1V_{0,2}As_1Cu_{0,2}Sb_{0,1}O_x$,

$Mo_{12}P_{1,0}Cs_{1,0}V_{0,2}As_{1,0}Cu_{0,2}O_x$,

$Mo_{12}P_{1,0}Cs_{1,0}V_{0,2}As_{1,5}Cu_{0,2}O_x$,

$Mo_{12}P_{1,15}Cs_{1,25}V_{0,05}As_{1,3}Cu_{0,3}O_x$,

$Mo_{12}P_{1,0}Cs_{1,0}V_{0,2}As_{1,6}Cu_{0,2}O_x$,

$Mo_{12}P_{1,1}Cs_{1,12}V_{0,3}As_{0,85}Cu_{0,2}Sb_{0,15}O_x$,

$Mo_{12}P_{1,0}Cs_{1,0}V_{0,2}As_{1,4}Cu_{0,2}O_x$,

$Mo_{12}P_{1,2}Cs_{1,25}V_{0,4}As_{0,7}Sb_{0,2}O_x$,

$Mo_{12}P_{1,2}Cs_{1,25}V_{0,2}As_{1,0}Cu_{0,2}Sb_{0,2}O_x$,

$Mo_{12}P_{1,15}Cs_{0,75}V_{0,35}As_{1,3}Cu_{0,3}O_x$,

$Mo_{12}P_{1,2}Cs_{1,25}V_{0,6}As_{0,5}Sb_{0,2}O_x$, et

$Mo_{12}P_{1,2}Cs_{1,25}V_{0,6}As_{0,7}Cu_{0,2}Sb_{0,2}O_x$.

6. Une composition catalytique comprenant un catalyseur comme défini dans l'une quelconque des revendications précédentes sous une forme fixée à un support.

7. Une composition comme revendiqué dans la revendication 6 dans laquelle le support est un des suivants: alumine, pierre ponce, carbure de silicium, zircone, silice, alumine-silice et perlite, de préférence alumine-silice.

8. Un procédé pour préparer un catalyseur utile soit dans un procédé comme revendiqué dans l'une quelconque des revendications 1 à 5, soit pour former une composition catalytique comme revendiqué dans la revendication 6 ou 7, lequel procédé comprend (a) la formation d'une suspension de trioxyde de molybdène dans l'eau, l'addition d'acide phosphorique à ladite suspension, le chauffage de la suspension obtenue entre 60° et 95°C pendant un temps au moins suffisant pour former de l'acide phosphomolybdique, puis l'addition d'hydroxyde d'ammonium, les éléments restants nécessaires ou acceptables étant ajoutés à la suspension sous forme d'oxydes ou de sels ou de composés convertibles par chauffage en les oxydes correspondants, soit avant, soit après l'addition de l'acide phosphorique pour former un précurseur de catalyseur,
(b) isolement du précurseur de catalyseur, et
(c) la calcination du précurseur isolé à une température de 350° à 450°C.

9. Un procédé comme revendiqué dans la revendication 8 dans lequel la suspension formée dans le stade (a) est chauffée pendant au moins 1 heure, de préférence au moins 3 heures.

10. Un procédé comme revendiqué dans la revendication 8 ou 9 dans lequel ledit précurseur est calciné à une température de 385° à 415°C de préférence d'environ 400°C.

11. Un procédé comme revendiqué dans l'une quelconque des revendications 1 à 5 dans lequel le catalyseur est une composition catalytique comme revendiqué dans la revendication 6 ou 7 ou un catalyseur préparé selon un procédé comme revendiqué dans la revendication 8 ou 9.